# EUROPEAN PATENT SPECIFICATION

(11) **EP 3 610 824 B1**
(45) Date of publication and mention of the grant of the patent: **24.11.2021**
(21) Application number: 18189013.8
(22) Date of filing: 14.08.2018
(51) Int. Cl.: A61C 5/73, A61C 13/00

(54) **DENTAL PROSTHESIS**
ZAHNPROTHESE
PROTHÈSE DENTAIRE

(43) Date of publication of application: 19.02.2020
(73) Proprietor: DENTSPLY SIRONA Inc., York, PA 17401 (US); DeguDent GmbH, 63457 Hanau (DE)
(72) Inventor: VOLLMANN, Markus, 63571 Gelnhausen (DE)
(74) Representative: Dietz, Mirko

(56) References cited:
- WO-A1-2014/028505
- WO-A1-2015/168463
- WO-A1-2017/149262
- KR-B1- 101 676 343
- US-A1- 2010 028 835

## Description

The invention relates to a dental prosthesis, in particular a crown, consisting of or comprising an inner primary part, such as a cap, that is connected in a fixed manner to an outer secondary part, such as a veneer.

The invention further relates to a dental kit for manufacturing or producing a dental prosthesis.

Usual dental prostheses, in particular crowns, consist of a framework of a metal as the primary part and a veneer covering the framework as the secondary part. This may be of ceramic. The material characteristics of the primary part and the secondary part are such that the modulus of elasticity of the primary part is greater than that of the secondary part. Dental frameworks of zirconium oxide, aluminum oxide, zirconium mixed oxide, aluminum mixed oxide or combinations thereof are also known (DE 103 69 319 C5). The dental framework can be coated by hot-pressing a lithium silicate blank onto the framework.

WO 2017/149262 A1 discloses a dental prosthesis, and a dental kit according to the preamble of claims 1 and 7, respectively.

An object underlying the present invention is to further develop a dental prosthesis of the type described above such that it has characteristics that correspond to those of a natural tooth.

A further object is to further develop a dental kit of the type described above such that the manufactured or produced dental prosthesis has characteristics that correspond to those of a natural tooth.

The present invention is set out in the appended claims 1 and 7. Preferred embodiments are set out in the dependent claims.

The object of the invention is substantially achieved in that the primary part consists of a plastic material or comprises such a material, that the secondary part comprises of a ceramic material or contains such a material, and that the primary part consists of material that has a modulus of elasticity E_{P} and the secondary part consists of material that has a modulus of elasticity Es where Ep < Es.

In particular, the modulus of elasticity E_{P} of the primary part is in the range 5 GPa to 30 GPa, particularly preferably in the range 10 GPa to 25 GPa. The secondary part, with the higher modulus of elasticity Es in particular has a value in the range 40 GPa to 120 GPa, and preferably in the range 50 GPa to 75 GPa.

In accordance with the invention and by way of departure from the known prior art, wherein the primary part has a greater modulus of elasticity than the secondary part, a biomimetic dental prosthesis is provided which thus has the characteristics of a natural tooth.

It is here in particular provided that the primary part consists of or comprises at least one material from the group polymethyl methacrylate (PMMA), in particular highly-filled PMMA, polyether ether ketone (PEEK), triethylene glycol dimethacrylate (TEGDMA), diethylene glycol dimethacrylate (DEGMA), urethane dimethacrylate (UDMA) or a combination or part-combination thereof.

It is also possible to use one or more of the above-mentioned materials as the primary material, to which one or more ceramic fillers such as quartz (SiO₂) or other glass ceramic fillers or oxide ceramic fillers have been added.

The primary part can also have a skeleton-like structure, for instance of a ceramic that is infiltrated with polymers.

A lithium silicate glass ceramic which is favored in particular is one which contains lithium disilicate and/or lithium metasilicate as the crystal phases.

Other ceramics, such as those based on phlogopite or glass ceramics from the LAS group (lithium oxide, aluminum oxide and/or silicon dioxide) or glass ceramics with virgilite crystals and/or sogdianite crystals may also be used.

The secondary part consists of or comprises a ceramic material, in particular a material from the group zirconium dioxide, in particular yttrium-stabilized zirconium dioxide, aluminum oxide, feldspar, lithium silicate glass ceramic or a mixture of one or more of these materials.

The secondary part consists of or includes as its starting material the following in % by weight:

| | |
|---|---|
| SiO₂ | 46.0-72.0 |
| Li₂O | 10.0-25.0 |
| ZrO₂ | 8.0-20.0 |
| Al₂O₃ | 0.1-8.0 |
| K₂O | 0.1-5.0 |
| CeO₂ | 0.0-4.0 |
| B₂O₃ | 0.0-4.0 |
| Na₂O | 0.0-4.0 |
| Tb₄O₇ | 0.0-2.5 |

at least one nucleating agent 1.0-10.0, such as P₂O₅,
as well as 0.0 to 4.0 of at least one additive,
where the entire sum is 100% by weight.

The secondary part preferably has a starting composition of, or includes, the following in % by weight:

| | |
|---|---|
| SiO₂ | 58-60 |
| Li₂O | 13.5-20.5 |
| ZrO₂ | 9.0-12.5 |
| P₂O₅ | 3.0-7.5 |
| Al₂O₃ | 0.5-6.0 |
| K₂O | 0.5-3.5 |
| CeO₂ | 0.5-2.5 |
| B₂O₃ | 0-3 |
| Na₂O | 0-3 |
| Tb₄O₇ | 0-1.5. |

The primary part can be manufactured from one block of plastic material in a CAD / CAM process. However, a thermoforming or deep drawing process is also possible.

The secondary part too can be manufactured in a CAD / CAM process. Other suitable manufacturing processes that are used to manufacture dental prostheses, such as pressing or additive manufacturing processes, can also be used.

In particular, the secondary part is connected to the primary part by bonding with adhesive or by cementing.

The usual dental polymer-based adhesives can be used as materials for the adhesive. These include non-curing and/or self-curing full adhesives or self-adhesives. Examples include dimethacrylate resins or bis-GMA (bisphenol A-glycidyl methacrylate).

Subject matter of the invention is also a dental kit for manufacturing or producing a dental prosthesis, in particular crown, consisting of or comprising an inner primary part, such as a cap, which may be joined in a fixed manner to an outer secondary part, wherein the dental kit comprises:
a) a first block for forming, using a CAD/CAM process, an inner primary part, wherein the first block consists of or comprises plastic material, where the material of the first block has a modulus of elasticity Ep;
b) a second block for forming, using a CAD/CAM process, an outer secondary part, wherein the second block consists of or comprises ceramic material, where the material of the second block has a modulus of elasticity Es where Ep < Es; and
c) optionally, one or more adhesive bonding agents suitable for joining the inner primary part in a fixed manner to the outer secondary part.

The modulus of elasticity E_{P} of the first block is in the range 5 GPa to 30 GPa, particularly preferably in the range 10 GPa to 25 GPa. The second block, with the higher modulus of elasticity Es has a value in the range 40 GPa to 120 GPa, and preferably in the range 50 GPa to 75 GPa.

It is here in particular provided that the first block consists of or includes at least one material from the group polymethyl methacrylate (PMMA), in particular highly-filled PMMA, polyether ether ketone (PEEK), triethylene glycol dimethacrylate (TEGDMA), diethylene glycol dimethacrylate (DEGMA), urethane dimethacrylate (UDMA) or a combination or part-combination thereof.

It is also possible to use one or more of the above-mentioned materials for the first block, to which one or more ceramic fillers such as quartz (SiO₂) or other glass ceramic fillers or oxide ceramic fillers have been added.

A lithium silicate glass ceramic which is favored in particular is one which contains lithium disilicate and/or lithium metasilicate as the crystal phases.

Other ceramics, such as those based on phlogopite or glass ceramics from the LAS group (lithium oxide, aluminum oxide and/or silicon dioxide) or glass ceramics with virgilite crystals and/or sogdianite crystals may also be used.

The second block consists of or includes a ceramic material, in particular a material from the group zirconium dioxide, in particular yttrium-stabilized zirconium dioxide, aluminum oxide, feldspar, lithium silicate glass ceramic or a mixture of one or more of these materials.

The second block advantageously consists of or comprises as its starting material the following in % by weight:

| | |
|---|---|
| SiO₂ | 46.0-72.0 |
| Li₂O | 10.0-25.0 |
| ZrO₂ | 8.0-20.0 |
| Al₂O₃ | 0.1-8.0 |
| K₂O | 0.1-5.0 |
| CeO₂ | 0.0-4.0 |

| | |
|---|---|
| B₂O₃ | 0.0-4.0 |
| Na₂O | 0.0-4.0 |
| Tb₄O₇ | 0.0-2.5 |

at least one nucleating agent 1.0-10.0, such as P₂O₅,
as well as 0.0 to 4.0 of at least one additive,
where the entire sum is 100% by weight.

The second block preferably has a starting composition of, or includes, the following in % by weight:

| | |
|---|---|
| SiO₂ | 58-60 |
| Li₂O | 13.5-20.5 |
| ZrO₂ | 9.0-12.5 |
| P₂O₅ | 3.0-7.5 |
| Al₂O₃ | 0.5-6.0 |
| K₂O | 0.5-3.5 |
| CeO₂ | 0.5-2.5 |
| B₂O₃ | 0-3 |
| Na₂O | 0-3 |
| Tb₄O₇ | 0-1.5. |

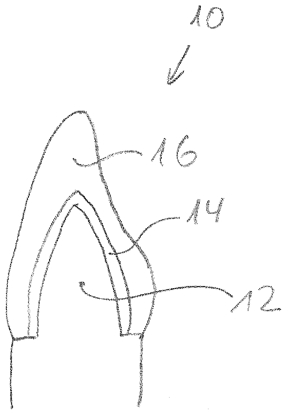

Further details, advantages and characteristics of the invention are provided not just from the claims and the characteristics to be drawn therefrom - singly and/or in combination -, but also from the following description of an embodiment shown in the drawing, as well as an example.

The teaching according to the invention is explained with reference to a crown 10, as shown in the single Figure, although no restriction results from this. Rather, the invention generally applies to any dental prosthesis that consists of a primary and secondary part, i.e. in the case of a crown a lower and an upper crown. Further examples include partial crowns, inlays, onlays, veneers and bridges, especially bridges that have three elements.

The Figure shows a prepared tooth stump 12. Taking the external shape of the tooth stump 12 into consideration, a cap 14 is first manufactured as the primary part and consists of or comprises plastic. Preferably the primary part consists of plastic. Preferred materials are high-density PPMA, UDMA, PEEK, DEGMA or TEGDMA or a mixture of one or more of these materials. Corresponding plastics with fill additives such as SiO₂ can also be used. The modulus of elasticity of the material used lies in the range 5 to 30 GPa.

The cap 14 can be manufactured from a plastic block using a CAD / CAM process, without any restriction being intended by this.

After manufacture of the cap 14, taking into consideration its outer geometry, the teeth adjoining the tooth stump and the antagonists, a veneer 16 is manufactured as secondary part in a CAD / CAM process from a block of lithium silicate glass ceramic or another suitable ceramic material that has a modulus of elasticity between 40 and 120 GPa. After cementing of the cap 14 to the stump 12, the secondary part 16 is bonded to the primary part. It can also be cemented.

By way of departure from a usual dental prosthesis, which consists of a framework of metal and a veneer, a biomimetic dental prosthesis is provided, in which the modulus of elasticity of the primary part corresponds to that of the dentine material and that of the secondary part corresponds to that of the dental enamel material, where the strength may be selected to be higher if necessary.

To permit conclusions about the working life of a corresponding dental prosthesis, artificial ageing was performed with a chewing simulator (Chewing Simulator CS-4.8 with TC4, made by SDM Mechatronic GmbH, Feldkirchen, Germany). For this purpose, ten crowns were manufactured, with the material for the primary part being highly-filled PMMA with a modulus of elasticity of 13 GPa. The primary part was manufactured from a PMMA block in a CAD / CAM process.

The secondary part was manufactured from a lithium disilicate glass ceramic block in a CAD / CAM process. The material used was a lithium disilicate glass ceramic marketed under the name CELTRA Duo by DeguDent GmbH, Hanau, Germany.

The crowns so manufactured were bonded to metal stumps with an adhesive material marketed under the name Multilink Hybrid Abutment. The ten crowns were each subjected to 1.2 million chewing cycles (1 Hz) together with 6,000 thermal cycles at 5 °C / 50 °C in water. Following chewing simulation, there were no cracks, fissures or delaminations detected. The mean breaking load of the ten crowns after ageing was 4,437 N +/- 1,012 N.

## Claims

1. A dental prosthesis (10), in particular crown, consisting of or comprising an inner primary part (14), such as a cap, which is joined in a fixed manner to an outer secondary part (16),
wherein the primary part (14) consists of or includes plastic material, that the secondary part consists of ceramic material, and that the primary part consists of material that has a modulus of elasticity E_{P} and the secondary part (16) consists of material that has a modulus of elasticity Es where Ep < Es; wherein the modulus of elasticity Ep of the primary part (14) is 5 GPa ≤ E_{P} ≤ 30 GPa, and/or the modulus of elasticity Es of the secondary part (16) is 40 GPa ≤ Es ≤ 120 GPa; wherein the primary part (14) consists of or includes at least one material from the group PMMA (polymethyl methacrylate), in particular highly-filled PMMA, PEEK (polyether ether ketone), TEGDMA (triethylene glycol dimethacrylate), DEGMA (diethylene glycol dimethacrylate), UDMA (urethane dimethacrylate) or a combination or part-combination thereof; and wherein the secondary part (16) consists of a starting material with the following composition in % by weight:
| | |
|---|---|
| SiO₂ | 46.0-72.0 |
| Li₂O | 10.0-25.0 |
| ZrO₂ | 8.0-20.0 |
| Al₂O₃ | 0.1-8.0 |
| K₂O | 0.1-5.0 |
| CeO₂ | 0.0-4.0 |
| B₂O₃ | 0.0-4.0 |
| Na₂O | 0.0-4.0 |
| Tb₄O₇ | 0.0-2.5 |
at least one nucleating agent 1.0-10.0, such as P₂O₅,
as well as 0.0 to 4.0 of at least one additive,
where the entire sum is 100% by weight.

2. The dental prosthesis according to claim 1,
**characterized in that**
the modulus of elasticity E_{P} of the primary part (14) is 10 GPa ≤ E_{P} ≤ 25 GPa, and/or the modulus of elasticity Es of the secondary part (16) is 50 GPa ≤ E_{S} ≤ 75 GPa.

3. The dental prosthesis according to claim 1 or 2,
**characterized in that**
the primary part (14) consists of or includes at least one material to which a ceramic-based fill material such as SiO₂, feldspar or zirconium dioxide has been additionally added.

4. The dental prosthesis according to one of the preceding claims,
**characterized in that**
the primary part (14) has a skeleton structure infiltrated with at least one polymer, said structure in particular consists of or contains a ceramic material.

5. The dental prosthesis according to at least one of the preceding claims,
**characterized in that**
the primary part (14) is connected to the secondary part (16) by bonding with adhesive or by cementing, wherein the adhesive material preferably consists of or includes at least one material from the group of dental polymer-based adhesives.

6. The dental prosthesis according to at least one of the preceding claims,
**characterized in that**
the secondary part (16) consists of a starting material with the following composition in % by weight:
| | |
|---|---|
| SiO₂ | 58-60 |
| Li₂O | 13.5-20.5 |
| ZrO₂ | 9.0-12.5 |
| P₂O₅ | 3.0-7.5 |
| Al₂O₃ | 0.5-6.0 |
| K₂O | 0.5-3.5 |
| CeO₂ | 0.5-2.5 |
| B₂O₃ | 0-3 |
| Na₂O | 0-3 |
| Tb₄O₇ | 0-1.5. |

7. A dental kit for manufacturing or producing a dental prosthesis (10), in particular crown, consisting of or comprising an inner primary part (14), such as a cap, which may be joined in a fixed manner to an outer secondary part (16), wherein the dental kit comprises:
a) a first block for forming, using a CAD/CAM process, the inner primary part (14), wherein the first block consists of or includes plastic material, where the material of the first block has a modulus of elasticity Ep;
b) a second block for forming, using a CAD/CAM process, the outer secondary part (16), wherein the second block consists of ceramic material, where the material of the second block has a modulus of elasticity Es where Ep < Es; and
c) optionally, one or more adhesive bonding agents suitable for joining the inner primary part (14) in a fixed manner to the outer secondary part (16);
wherein the modulus of elasticity EP of the material of the first block is 5 GPa ≤ EP ≤ 30 GPa, and/or the modulus of elasticity ES of the material of the second block is 40 GPa ≤ ES ≤ 120 GPa;
wherein the first block consists of or includes at least one material from the group PMMA (polymethyl methacrylate), PEEK (polyether ether ketone), TEGDMA (triethylene glycol dimethacrylate), DEGMA (diethylene glycol dimethacrylate), UDMA (urethane dimethacrylate) or a combination or part-combination thereof; and
wherein the secondary part (16) consists of a starting material with the following composition in % by weight:
| | |
|---|---|
| SiO₂ | 46.0-72.0 |
| Li₂O | 10.0-25.0 |
| ZrO₂ | 8.0-20.0 |
| Al₂O₃ | 0.1-8.0 |
| K₂O | 0.1-5.0 |
| CeO₂ | 0.0-4.0 |
| B₂O₃ | 0.0-4.0 |
| Na₂O | 0.0-4.0 |
| Tb₄O₇ | 0.0-2.5 |
at least one nucleating agent 1.0-10.0, such as P₂O₅,
as well as 0.0 to 4.0 of at least one additive,
where the entire sum is 100% by weight.

8. The dental kit according to claim 7,
**characterized in that**
the modulus of elasticity Ep of the material of the first block is 10 GPa ≤ Ep ≤ 25 GPa, and/or the modulus of elasticity Es of the material of the second block is 50 GPa ≤ Es ≤ 75 GPa.

9. The dental kit according to claim 7 or 8,
**characterized in that**
the first block consists of or includes at least one material to which a ceramic-based fill material such as SiO₂, feldspar or zirconium dioxide has been additionally added.

10. The dental kit according to one of the preceding claims 7 to 9,
**characterized in that**
the second block consists of a starting material with the following composition in % by weight:
| | |
|---|---|
| SiO₂ | 58-60 |
| Li₂O | 13.5-20.5 |
| ZrO₂ | 9.0-12.5 |
| P₂O₅ | 3.0-7.5 |
| Al₂O₃ | 0.5-6.0 |
| K₂O | 0.5-3.5 |
| CeO₂ | 0.5-2.5 |
| B₂O₃ | 0-3 |
| Na₂O | 0-3 |
| Tb₄O₇ | 0-1.5. |

## Patentansprüche

1. Zahnprothese (10), insbesondere Krone, bestehend aus oder umfassend ein inneres Primärteil (14), wie etwa eine Kappe, das mit einem äußeren Sekundärteil (16) fest verbunden ist, wobei das Primärteil (14) aus Kunststoffmaterial besteht oder dieses einschließt, dass das Sekundärteil aus Keramikmaterial besteht, und dass das Primärteil aus Material besteht, das ein Elastizitätsmodul Ep aufweist und das Sekundärteil (16) aus Material besteht, das ein Elastizitätsmodul E_{S} aufweist, wobei E_{P}< E_{S} ist; wobei das Elastizitätsmodul E_{P} des Primärteils (14) 5 GPa ≤ E_{P} ≤ 30 GPa beträgt, und/oder das Elastizitätsmodul E_{S} des Sekundärteils (16) 40 GPa ≤ E_{S} ≤ 120 GPa beträgt; wobei das Primärteil (14) aus mindestens einem Material aus der Gruppe PMMA (Polymethylmethacrylat), insbesondere hochgefülltes PMMA, PEEK (Polyetheretherketon), TEGDMA (Triethylenglykoldimethacrylat), DEGMA (Diethylenglykoldimethacrylat), UDMA (Urethandimethacrylat) oder einer Kombination oder Teilkombination davon besteht oder diese einschließt; und wobei das Sekundärteil (16) aus einem Ausgangsmaterial mit der folgenden Zusammensetzung in Gew.-% besteht:
| | |
|---|---|
| SiO₂ | 46,0-72,0 |
| Li₂O | 10,0-25,0 |
| ZrO₂ | 8,0-20,0 |
| Al₂O₃ | 0,1-8,0 |
| K₂O | 0,1-5,0 |
| CeO₂ | 0,0-4,0 |
| B₂O₃ | 0,0-4,0 |
| Na₂O | 0,0-4,0 |
| Tb₄O₇ | 0,0-2,5 |
mindestens ein Nukleierungsmittel 1,0-10,0, wie etwa P₂O₅,
sowie 0,0 bis 4,0 mindestens eines Additivs,
wobei die Gesamtsumme 100 Gew.-% beträgt.

2. Zahnprothese nach Anspruch 1,
**dadurch gekennzeichnet, dass**
das Elastizitätsmodul Ep des Primärteils (14) 10 GPa ≤ E_{P} ≤ 25 GPa beträgt, und/oder das Elastizitätsmodul Es des Sekundärteils (16) 50 GPa ≤ E_{S} ≤ 75 GPa beträgt.

3. Zahnprothese nach Anspruch 1 oder 2,
**dadurch gekennzeichnet, dass**
das Primärteil (14) aus mindestens einem Material besteht oder dieses einschließt, dem zusätzlich ein Füllmaterial auf Keramikbasis, wie etwa SiO₂, Feldspat oder Zirkondioxid, zugesetzt ist.

4. Zahnprothese nach einem der vorstehenden Ansprüche,
**dadurch gekennzeichnet, dass**
das Primärteil (14) eine mit mindestens einem Polymer infiltrierte Skelettstruktur aufweist, die insbesondere aus einem Keramikmaterial besteht oder dieses enthält.

5. Zahnprothese nach mindestens einem der vorstehenden Ansprüche,
**dadurch gekennzeichnet, dass**
das Primärteil (14) mit dem Sekundärteil (16) durch Kleben oder durch Zementieren verbunden ist, wobei das Klebematerial vorzugsweise aus mindestens einem Material aus der Gruppe der dentalpolymerbasierten Adhäsive besteht oder dieses einschließt.

6. Zahnprothese nach mindestens einem der vorstehenden Ansprüche,
**dadurch gekennzeichnet, dass**
das Sekundärteil (16) aus einem Ausgangsmaterial mit folgender Zusammensetzung in Gew.-% besteht:
| | |
|---|---|
| SiO₂ | 58-60 |
| Li₂O | 13,5-20,5 |
| ZrO₂ | 9,0-12,5 |
| P₂O₅ | 3,0-7,5 |
| Al₂O₃ | 0,5-6,0 |
| K₂O | 0,5-3,5 |
| CeO₂ | 0,5-2,5 |
| B₂O₃ | 0-3 |
| Na₂O | 0-3 |
| Tb₄O₇ | 0-1,5. |

7. Zahnkit zur Herstellung oder Produktion einer Zahnprothese (10), insbesondere Krone, bestehend aus oder umfassend ein inneres Primärteil (14), wie etwa eine Kappe, das mit einem äußeren Sekundärteil (16) fest verbunden werden kann, wobei das Zahnkit umfasst:
a) einen ersten Block zum Bilden des inneren Primärteils (14) unter Verwendung eines CAD/CAM-Verfahrens, wobei der erste Block aus Kunststoffmaterial besteht oder dieses einschließt, wobei das Material des ersten Blocks ein Elastizitätsmodul Ep aufweist;
b) einen zweiten Block zum Bilden des äußeren Sekundärteils (16) unter Verwendung eines CAD/CAM-Verfahrens, wobei der zweite Block aus Keramikmaterial besteht, wobei das Material des zweiten Blocks ein Elastizitätsmodul Es aufweist, wobei Ep < Es ist; und
c) optional ein oder mehrere Klebemittel, die geeignet sind, das innere Primärteil (14) fest mit dem äußeren Sekundärteil (16) zu verbinden;
wobei das Elastizitätsmodul EP des Materials des ersten Blocks 5 GPa ≤ EP ≤ 30 GPa beträgt und/oder das Elastizitätsmodul ES des Materials des zweiten Blocks 40 GPa ≤ ES ≤ 120 GPa beträgt;
wobei der erste Block aus mindestens einem Material aus der Gruppe PMMA (Polymethylmethacrylat), PEEK (Polyetheretherketon), TEGDMA (Triethylenglykoldimethacrylat), DEGMA (Diethylenglykoldimethacrylat), UDMA (Urethandimethacrylat) oder einer Kombination oder einer Teilkombination davon besteht oder diese einschließt; und
wobei das Sekundärteil (16) aus einem Ausgangsmaterial mit folgender Zusammensetzung in Gew.-% besteht:
| | |
|---|---|
| SiO₂ | 46,0-72,0 |
| Li₂O | 10,0-25,0 |
| ZrO₂ | 8,0-20,0 |
| Al₂O₃ | 0,1-8,0 |
| K₂O | 0,1-5,0 |
| CeO₂ | 0,0-4,0 |
| B₂O₃ | 0,0-4,0 |
| Na₂O | 0,0-4,0 |
| Tb₄O₇ | 0,0-2,5 |
mindestens ein Nukleierungsmittel 1,0-10,0, wie etwa P₂O₅,
sowie 0,0 bis 4,0 mindestens eines Additivs,
wobei die Gesamtsumme 100 Gew.-% beträgt.

8. Zahnkit nach Anspruch 7,
**dadurch gekennzeichnet, dass**
das Elastizitätsmodul E_{P} des Materials des ersten Blocks 10 GPa ≤ E_{P} ≤ 25 GPa beträgt und/oder das Elastizitätsmodul Es des Materials des zweiten Blocks 50 GPa ≤ Es ≤ 75 GPa beträgt.

9. Zahnkit nach Anspruch 7 oder 8,
**dadurch gekennzeichnet, dass**
der erste Block aus mindestens einem Material besteht oder dieses einschließt, dem zusätzlich ein Füllmaterial auf Keramikbasis, wie etwa SiO₂, Feldspat oder Zirkondioxid, zugesetzt ist.

10. Zahnkit nach einem der vorstehenden Ansprüche 7 bis 9,
**dadurch gekennzeichnet, dass**
der zweite Block aus einem Ausgangsmaterial mit folgender Zusammensetzung in Gew.-% besteht:
| | |
|---|---|
| SiO₂ | 58-60 |
| Li₂O | 13,5-20,5 |
| ZrO₂ | 9,0-12,5 |
| P₂O₅ | 3,0-7,5 |
| Al₂O₃ | 0,5-6,0 |
| K₂O | 0,5-3,5 |
| CeO₂ | 0,5-2,5 |
| B₂O₃ | 0-3 |
| Na₂O | 0-3 |
| Tb₄O₇ | 0-1,5. |

## Revendications

1. Prothèse dentaire (10), en particulier couronne, constituée de ou comprenant une partie primaire (14) interne, telle qu'un capuchon, lequel est relié de manière fixe à une partie secondaire (16) externe, dans laquelle la partie primaire (14) est constituée de ou comprend une matière plastique, la partie secondaire est constituée d'une matière céramique et la partie primaire est constituée d'une matière présentant un module d'élasticité E_{P} et la partie secondaire (16) est constituée d'une matière présentant un module d'élasticité E_{S} où E_{P} < E_{S} ; dans laquelle le module d'élasticité E_{P} de la partie primaire (14) est tel que 5 GPa ≤ E_{P} ≤ 30 GPa, et/ou le module d'élasticité E_{S} de la partie secondaire (16) est tel que 40 GPa ≤ E_{S} ≤ 120 GPa ; dans laquelle la partie primaire (14) est constituée de ou comprend au moins une matière du groupe PMMA (poly(méthacrylate de méthyle)), en particulier PMMA fortement chargé, PEEK (polyétheréthercétone), TEGDMA (diméthacrylate de triéthylèneglycol), DEGMA (diméthacrylate de diéthylèneglycol), UDMA (diméthacrylate d'uréthane) ou une combinaison ou une combinaison partielle de ceux-ci ; et dans laquelle la partie secondaire (16) est constituée d'une matière de départ présentant la composition suivante en % en poids :
| | |
|---|---|
| SiO₂ | 46,0 - 72,0 |
| Li₂O | 10,0-25,0 |
| ZrO₂ | 8,0 - 20,0 |
| Al₂O₃ | 0,1-8,0 |
| K₂O | 0,1-5,0 |
| CeO₂ | 0,0 - 4,0 |
| B₂O₃ | 0,0 - 4,0 |
| Na₂O | 0,0-4,0 |
| Tb₄O₇ | 0,0 - 2,5 |
au moins un agent de nucléation 1,0 - 10,0, tel que P₂O₅,
ainsi que 0,0 à 4,0 d'au moins un additif,
la somme totale étant de 100 % en poids.

2. Prothèse dentaire selon la revendication 1,
**caractérisée en ce que**
le module d'élasticité E_{P} de la partie primaire (14) est tel que 10 GPa ≤ E_{P} ≤ 25 GPa, et/ou le module d'élasticité Es de la partie secondaire (16) est tel que 50 GPa ≤ Es ≤ 75 GPa.

3. Prothèse dentaire selon la revendication 1 ou 2,
**caractérisée en ce que**
la partie primaire (14) est constituée de ou comprend au moins une matière à laquelle a été ajoutée en plus une matière de remplissage à base de céramique telle que SiO2, le feldspath ou le dioxyde de zirconium.

4. Prothèse dentaire selon l'une quelconque des revendications précédentes,
**caractérisée en ce que**
la partie primaire (14) présente une structure squelette infiltrée d'au moins un polymère, ladite structure étant en particulier constituée de ou contenant une matière céramique.

5. Prothèse dentaire selon au moins l'une quelconque des revendications précédentes,
**caractérisée en ce que**
la partie primaire (14) est reliée à la partie secondaire (16) par liaison à l'aide d'un adhésif ou par cémentation, la matière adhésive étant de préférence constituée de ou comprenant au moins une matière du groupe des colles dentaires à base de polymères.

6. Prothèse dentaire selon au moins l'une quelconque des revendications précédentes,
**caractérisée en ce que**
la partie secondaire (16) est constituée d'une matière première présentant la composition suivante en % en poids :
| | |
|---|---|
| SiO₂ | 58 - 60 |
| Li₂O | 13,5 - 20,5 |
| ZrO₂ | 9,0 - 12,5 |
| P₂O₅ | 3,0 - 7,5 |
| Al₂O₃ | 0,5 - 6,0 |
| K₂O | 0,5 - 3,5 |
| CeO₂ | 0,5 - 2,5 |
| B₂O₃ | 0 - 3 |
| Na₂O | 0 - 3 |
| Tb₄O₇ | 0 - 1,5. |

7. Kit dentaire destiné à la fabrication ou la production d'une prothèse dentaire (10), en particulier d'une couronne, constituée de ou comprenant une partie primaire (14) interne, telle qu'un capuchon, lequel peut être relié de manière fixe à une partie secondaire (16) externe, le kit dentaire comprenant :
a) un premier bloc permettant de former, à l'aide d'un procédé CAD/CAM, la partie primaire (14) interne, dans lequel le premier bloc est constitué de ou comprend une matière plastique, la matière du premier bloc présentant un module d'élasticité Ep;
b) un second bloc permettant de former, à l'aide d'un procédé CAD/CAM, la partie secondaire (16) externe, dans lequel le second bloc est constitué de matière céramique, la matière du second bloc présentant un module d'élasticité Es où Ep < Es ; et
c) éventuellement, au moins un agent de liaison à l'aide d'un adhésif apte à la liaison de la partie primaire (14) interne de manière fixe à la partie secondaire (16) externe ;
dans lequel le module d'élasticité EP de la matière du premier bloc est tel que 5 GPa ≤ EP ≤ 30 GPa, et/ou le module d'élasticité ES de la matière du second bloc est tel que 40 GPa ≤ ES ≤ 120 GPa ;
dans lequel le premier bloc est constitué de ou comprend au moins une matière du groupe PMMA (poly(méthacrylate de méthyle)), PEEK (polyétheréthercétone), TEGDMA (diméthacrylate de triéthylèneglycol), DEGMA (diméthacrylate de diéthylèneglycol), UDMA (diméthacrylate d'uréthane) ou une combinaison ou une combinaison partielle de ceux-ci ; et
dans lequel la partie secondaire (16) est constituée d'une matière première présentant la composition suivante en % en poids :
| | |
|---|---|
| SiO₂ | 46,0 - 72,0 |
| Li₂O | 10,0 - 25,0 |
| ZrO₂ | 8,0 - 20,0 |
| Al₂O₃ | 0,1 - 8,0 |
| K₂O | 0,1 - 5,0 |
| CeO₂ | 0,0 - 4,0 |
| B₂O₃ | 0,0 - 4,0 |
| Na₂O | 0,0 - 4,0 |
| Tb₄O₇ | 0,0 - 2,5 |
au moins un agent de nucléation 1,0 - 10,0, tel que P₂O₅,
ainsi que 0,0 à 4,0 d'au moins un additif,
la somme totale étant de 100 % en poids.

8. Kit dentaire selon la revendication 7,
**caractérisé en ce que**
le module d'élasticité E_{P} de la matière du premier bloc est tel que 10 GPa ≤ E_{P} ≤ 25 GPa, et/ou le module d'élasticité Es de la matière du second bloc est tel que 50 GPa ≤ Es ≤ 75 GPa.

9. Kit dentaire selon la revendication 7 ou 8,
**caractérisé en ce que**
le premier bloc est constitué de ou comprend au moins une matière à laquelle a été ajoutée en plus une matière de remplissage à base de céramique telle que SiO₂, le feldspath ou le dioxyde de zirconium.

10. Kit dentaire selon l'une quelconque des revendications 7 à 9,
**caractérisé en ce que**
le second bloc est constitué d'une matière première présentant la composition suivante en % en poids :
| | |
|---|---|
| SiO₂ | 58 - 60 |
| Li₂O | 13,5 - 20,5 |
| ZrO₂ | 9,0 - 12,5 |
| P₂O₅ | 3,0 - 7,5 |
| Al₂O₃ | 0,5 - 6,0 |
| K₂O | 0,5 - 3,5 |
| CeO₂ | 0,5 - 2,5 |
| B₂O₃ | 0 - 3 |
| Na₂O | 0 - 3 |
| Tb₄O₇ | 0 - 1,5. |
